# EUROPEAN PATENT SPECIFICATION

(11) **EP 4 327 801 B1**
(45) Date of publication and mention of the grant of the patent: **09.07.2025**
(21) Application number: 23171958.4
(22) Date of filing: 05.05.2023
(51) Int. Cl.: A61K 8/44, A61K 8/46, A61Q 19/10, A61Q 5/02, A61Q 5/12

(54) **SULFATE-FREE SHAMPOO**
SULFATFREIES SHAMPOO
SHAMPOOING SANS SULFATE

(30) Priority: 24.08.2022 EP 22191865
(43) Date of publication of application: 28.02.2024
(73) Proprietor: Wella Germany GmbH, 64295 Darmstadt (DE)
(72) Inventor: SPRINGOB, Christian, 64295 Darmstadt (DE)
(74) Representative: Baier, Martin

(56) References cited:
- WO-A1-2020/233972
- WO-A1-2022/005839
- CN-A- 101 375 826
- US-A1- 2015 157 548
- DATABASE GNPD [online] MINTEL; 27 June 2022 (2022-06-27), ANONYMOUS: "Coconut Water Buildup Removal Shampoo", XP093023979, retrieved from https://www.gnpd.com/sinatra/recordpage/9694792/ Database accession no. 9694792

## Description

### Technical Field

The presently claimed invention relates to a cleansing composition comprising at least one taurate surfactant; at least one surfactant selected from the group consisting of isethionate surfactants and methyl isethionate surfactants; at least one N-acylated amino acid surfactant; and at least one betaine which is used as a hair conditioning composition, a method for applying the cleansing composition to hair and the use of the cleansing composition for cleansing hair.

### Background

Hair shampoos and personal care cleansing products in general are typically formulated with anionic surfactants or with a combination of an anionic surfactant and one or more secondary surfactants selected from the other surfactant classes. Anionic surfactants are known for their excellent foaming properties which directly relate to the perceived shampoo efficiency of the composition. Especially sulfate-based surfactants, i.e., sodium lauryl sulfate and sodium lauryl ether sulfate, are often used in shampoo compositions because of their effectiveness in foam production. Nevertheless, sulfate-based surfactants can be harsh and irritating to skin and can contribute to color fading and drying of hair.

Eliminating sulfate surfactants from shampoo compositions remains challenging because sulfate-free compositions typically have poor foaming properties.

EP 3 416 613 A1 discloses a personal care composition containing a particular combination of anionic surfactants, one of which is a specific taurate and another one of which is one specific isethionate, together with a specific amphoteric or zwitterionic surfactant. The personal care composition delivers acceptable foaming and conditioning properties.

WO 2020/043336 A1 describes a composition comprising sodium lauroyl methyl isethionate, sodium methyl cocoyl taurate and cocoamidopropyl betaine.

EP 3 079 654 B1 discloses a personal care composition comprising a mixture of anionic surfactants, amphoteric surfactants, a cationic-substituted guar and a copolymer of acrylamidopropyltrimonium chloride.

WO 2022/005839 A1 discloses sulfate-free shampoo formulations that deliver cosmetic benefits to the hair, while also providing desirable performance and feel properties. The formulations do not contain at least one N-acylated amino acid surfactant selected from sarcosinates.

The methods and compositions disclosed in the prior arts have limitations.

There is a need for personal care cleansing products with good foaming properties which do not compromise on hair conditioning performance, while at the same time being free of sulfate-containing surfactants.

Hence, it is an object of the presently claimed invention to provide cleansing compositions that show good foaming properties and do not compromise on hair conditioning performance, while at the same time being free of sulfate-containing surfactants.

### Summary

Surprisingly, it was found that the addition of at least one N-acylated amino acid surfactant to a mixture of anionic surfactants and at least one betaine results in the formation of cleansing compositions that show good foaming properties and do not compromise on hair conditioning performance.

Accordingly, in one aspect, the presently claimed invention is directed to a cleansing composition comprising
(A) ≥1.0 to ≤ 8.0 wt.% of at least one taurate surfactant;
(B) ≥1.0 to ≤ 10.0 wt.% of at least one surfactant selected from the group consisting of isethionate surfactants and methyl isethionate surfactants;
(C) ≥0.5 to ≤ 5.0 wt.% of at least one N-acylated amino acid surfactant; and
(D) ≥1.0 to ≤8.0 wt.% of at least one betaine;

whereby the wt.% are each based on the total weight of the cleansing composition, and wherein
the (C) at least one N-acylated amino acid surfactant is selected from the group consisting of sodium lauroyl sarcosinate, sodium cocoyl sarcosinate, sodium myristoyl sarcosinate, sodium oleoyl sarcosinate, and ammonium lauroyl sarcosinate .

In another aspect, the presently claimed invention is directed to a method for cleansing hair comprising applying the cleansing composition of the presently claimed invention as described herein to hair.

In another aspect, the presently claimed invention is directed to the use of a cleansing composition of the presently claimed invention as described herein for cleansing hair.

Other objects, advantages and applications of the presently claimed invention will become apparent to those skilled in the art from the following detailed description.

### Detailed description

The following detailed description is merely exemplary in nature.

Furthermore, the ranges defined throughout the specification include the end values as well, i.e., a range of 1 to 10 implies that both 1 and 10 are included in the range. For the avoidance of doubt, the applicant shall be entitled to any equivalents according to applicable law.

For the purposes of the presently claimed invention, '% by weight' or 'wt.% 'as used in the presently claimed invention is with respect to the total weight of the cleansing composition. Further, the sum of wt.-% of all the compounds, as described herein, in the respective components adds up to 100 wt.-%.

For the purpose of the presently claimed invention, by the expression "foaming properties" it is meant flash foam and foam volume which are among the main factors affecting the consumer perception about the foam quality. Well-known tests, notably as described in the example part, may be used to measure these factors.

For the purpose of the presently claimed invention, by the expression "conditioning on hair" it is meant imparting positive properties to the hair. For example, "improved conditioning" may cover improved ease of detangling and/or ease of combing. Ease of detangling may be determined by the measurement of the time required for detangling the hair. The shorter the detangling time is, the easier the hair to detangle is. Ease of combing may be determined by the measurement of the work required for combing the hair. The lower the combing work is, the easier the hair to comb is.

The measurement techniques described hereinabove and herein are well known to a person skilled in the art .

An aspect of the presently claimed invention is directed a cleansing composition comprising
(A) ≥1.0 to ≤ 8.0 wt.% of at least one taurate surfactant;
(B) ≥1.0 to ≤ 10.0 wt.% of at least one surfactant selected from the group consisting of isethionate surfactants and methyl isethionate surfactants;
(C) ≥0.5 to ≤ 5.0 wt.% of at least one N-acylated amino acid surfactant; and
(D) ≥1.0 to ≤8.0 wt.% of at least one betaine;

whereby the wt.% are each based on the total weight of the cleansing composition, and
wherein the (C) at least one N-acylated amino acid surfactant is selected from the group consisting of sodium lauroyl sarcosinate, sodium cocoyl sarcosinate, sodium myristoyl sarcosinate, sodium oleoyl sarcosinate, and ammonium lauroyl sarcosinate .

In a preferred embodiment, the cleansing composition of the presently claimed invention comprises at least one taurate surfactant which is a compound of general formula (I),

R^{a}-C(=O)-N(CH₃)-CH₂-CH₂-SO₃-X (I),

wherein R^{a} is a hydrocarbon radical and X is a counterion.

The hydrocarbon radical R^{a} preferably contains 10 to 22 carbon atoms. The counterion X is preferably an alkali metal ion, alkaline earth metal ion or ammonium ion. The alkali metal ion is preferably a sodium ion. The alkali metal ion is preferably another alkali metal ion such as potassium or lithium. The alkaline earth metal ion is preferably calcium or magnesium. The ammonium ion is preferably an alkyl ammonium ion having up to 6 carbon atoms such as isopropylammonium.

In a preferred embodiment, the at least one taurate surfactant (A) is selected from the group consisting of sodium methyl lauroyl taurate, sodium methyl myristoyl taurate, potassium methyl myristoyl taurate, sodium methyl cocoyl taurate, sodium methyl oleoyl taurate, calcium methyl lauroyl taurate, potassium methyl lauroyl taurate and ammonium methyl lauroyl taurate, more preferably from the group consisting of sodium methyl cocoyl taurate and sodium methyl oleoyl taurate.

In a preferred embodiment, the at least one component (A) is present in the cleansing composition of the presently claimed invention in an amount in the range of ≥ 1.5 to ≤ 5.0 wt.%, more preferably in the range of ≥ 1.5 to ≤ 4.0 wt.%, even more preferably in the range of ≥ 2.0 to ≤ 3.0 wt.%, based on the total weight of the cleansing composition.

In a preferred embodiment, the cleansing composition of the presently claimed invention comprises at least one isethionate surfactant which is a compound of general formula (II),

R^{b}-C(=O)-O-CH₂-CH₂-SO₃-Y (II),

or at least one methyl isethionate surfactants which is a compound of general formula (III),

R^{b}-C(=O)-O-CH₂-CH(CH₃)-SO₃-Y (III),

wherein R^{b} is a substituted or unsubstituted alkyl, alkenyl, aryl or alkylaryl group having 6 to 30 carbon atoms and Y is a counterion, preferably R^{b} is an unsubstituted alkyl group having 6 to 30 carbon atoms, more preferably R^{b} is an unsubstituted alkyl group having 7 to 21 carbon atoms.

In a preferred embodiment, the at least one surfactant (B) comprises a mixture of fatty acids to form a mixture of isethionates of general formula (II) or methyl isethionates of formula (III), in which R^{b} is the same or different. Fatty acids are preferably obtained from natural oils that often include mixtures of fatty acids. For example, the fatty acid obtained from coconut oil contains a mixture of fatty acids including C12 lauric acid, C14 myristic acid, C16 palmitic acid and C8 caprylic acid.

In a preferred embodiment, R^{b} comprises the residue of one or more naturally occurring fatty acids or one or more synthetic fatty acids. Examples of carboxylic acids from which R^{b} is preferably derived include coco acid, hexanoic acid, caproic acid, caprylic acid, capric acid, lauric acid, myristic acid, palmitic acid, palmitoleic acid, stearic acid, oleic acid, linoleic acid, arachidic acid, gadoleic acid, arachidonic acid, eicosapentaenoic acid, behenic acid, erucic acid, docosahexaenoic acid, lignoceric acid, naturally occurring fatty acids such as those obtained from coconut oil, palm kernel oil, butterfat, palm oil, olive oil, corn oil, linseed oil, peanut oil, fish oil and rapeseed oil.

In a preferred embodiment, the counterion Y is an alkali metal ion, alkaline earth metal ion or ammonium ion. The alkali metal ion is preferably a sodium ion. The alkali metal ion is preferably another alkali metal ion such as potassium or lithium. The alkaline earth metal ion is preferably calcium or magnesium. The ammonium ion is preferably an alkyl ammonium ion having up to 6 carbon atoms such as isopropylammonium.

In a preferred embodiment, the at least one surfactant (B) is selected from the group consisting of sodium lauroyl isethionate, sodium lauroyl methyl isethionate, sodium myristoyl isethionate, sodium myristoyl methyl isethionate, sodium cocoyl isethionate, sodium cocoyl methyl isethionate, sodium cocoyl isethionate, sodium cocoyl methyl isethionate, ammonium oleoyl isethionate and ammonium oleoyl methyl isethionate, more preferably from the group consisting of sodium cocoyl isethionate and sodium cocoyl methyl isethionate.

In a preferred embodiment, the at least one component (B) is present in the cleansing composition of the presently claimed invention in an amount in the range of ≥ 1.5 or 2.0 to ≤ 8.0 wt.%, more preferably in the range of ≥ 3.0 to ≤ 7.0 wt.%, even more preferably in the range of ≥ 4.0 to ≤ 6.0 or 5.0 wt.% based on the total weight of the cleansing composition.

The at least one N-acylated amino acid surfactant (C) is selected from the group consisting of sodium lauroyl sarcosinate, sodium cocoyl sarcosinate, sodium myristoyl sarcosinate, sodium oleoyl sarcosinate, and ammonium lauroyl sarcosinate, more preferably the at least one N-acylated amino acid surfactant (C) is selected from the group consisting of sodium lauroyl sarcosinate.

In a preferred embodiment, the at least one component (C) is present in the cleansing composition of the presently claimed invention in an amount in the range of ≥ 1.0 to ≤ 4.0 wt.%, more preferably in the range of ≥ 1.0 to ≤ 3.0 wt.%, even more preferably in the range of ≥ 1.0 to ≤ 2.0 wt.%, based on the total weight of the cleansing composition.

In a preferred embodiment, the cleansing composition of the presently claimed invention comprises at least one betaine (D) which is selected from the group consisting of alkylbetaines, alkylamidopropylbetaines and alkylsulfobetaines.

In a preferred embodiment, the cleansing composition of the presently claimed invention comprises at least one alkylbetaine of general formula (V),

R^{f}R^{g}R^{h}N⁺-CH₂-C(=O)-O⁻ (V),

wherein R^{f} is a substituted or unsubstituted alkyl or alkenyl group having 7 to 22 carbon atoms and R^{g} and R^{h} are each, same or different, selected from the group consisting of C1-C6 alkyl, C1-C6 hydroxyalkyl and C1-C6 carboxyalkyl. R^{f} is preferably a mixture of C12 and C14 alkyl groups derived from coconut so that at least half, preferably at least three quarters, of the groups R^{f} have 10 to 14 carbon atoms. R^{g} and R^{h} are preferably methyl.

In a preferred embodiment, the at least one betaine (D) is selected from the group consisting of coco betaine, cocoamidopropyl betaine, lauryl betaine, laurylhydroxy sulfobetaine, lauryldimethyl betaine, cocoamidopropylhydroxysulfo betaine, behenyl betaine, capryl betaine, capramidopropyl betaine and stearyl betaine, more preferably the at least one betaine (D) is cocoamidopropyl betaine.

In a preferred embodiment, the at least one component (D) is present in the cleansing composition of the presently claimed invention in an amount in the range of ≥ 2.0 to ≤ 6.0 wt.%, more preferably in the range of ≥ 3.0 to ≤ 5.0 wt.%, even more preferably in the range of ≥ 4.0 to ≤ 5.0 wt.%, based on the total weight of the cleansing composition.

In a preferred embodiment, the cleansing composition of the presently claimed invention comprises water; more preferably the water is present in an amount in the range of ≥ 80.0 to ≤ 96.5 wt.%, based on the total weight of the cleansing composition. In another embodiment, the cleansing composition of the presently invention is present in the form of a concentrate that is suitable to prepare a composition of the presently claimed invention that contains water in an amount in the range of ≥ 80.0 to ≤ 96.5 wt.%, based on the total weight of the cleansing composition. Cleansing compositions are usually prepared by mixing the individual surfactants and conditioning agents. These components may be supplied as concentrated solutions which are diluted and/or combined in appropriate ratios by the formulator.

In a preferred embodiment, the cleansing composition of the presently claimed invention comprises at least one conditioning agent (E). The conditioning agent (E) is preferably a cationic or ampholytic conditioning agent. Conditioning agents assist in oil deposition. They preferably provide some conditioning effects They can, e.g., enhance the appearance and feel of hair, increase hair body or suppleness, facilitate combing and styling, improve gloss or sheen and improve the texture of hair that has been damaged by chemical or physical action. They can provide anti-static effect in altering the static electrical properties of hair.

In a preferred embodiment, the at least one conditioning agent (E) is a cationic cellulose. Cationic cellulose comprises salts of hydroxyethyl cellulose reacted with trimethyl ammonium substituted epoxide, referred to in the industry as polyquaternium-10. Another type of cationic cellulose includes the polymeric quaternary ammonium salts of hydroxyethyl cellulose reacted with lauryl dimethyl ammonium-substituted epoxide, referred to in the industry as polyquaternium-24.

In another preferred embodiment, the at least one conditioning agent (E) is a cationic polysaccharide polymer, especially a cationic guar gum derivative, such as guar hydroxypropyltrimonium chloride which is commercially available from Solvay in their JAGUAR trademark series.

In a preferred embodiment, the at least one component (E) is selected from the group consisting of polyquaternium-1, polyquaternium-2, polyquaternium-4, polyquaternium-5, polyquaternium-6, polyquaternium-7, polyquaternium-8, polyquaternium-9, polyquaternium-10, polyquaternium-11, polyquaternium-12, polyquaternium-13, polyquaternium-14, polyquaternium-15, polyquaternium-16, polyquaternium-17, polyquaternium-18, polyquaternium-19, polyquaternium-20, polyquaternium-22, polyquaternium-24, polyquaternium-27, polyquaternium-28, polyquaternium-29, polyquaternium-30, polyquaternium-31, polyquaternium-32, polyquaternium-33, polyquaternium-34, polyquaternium-35, polyquaternium-36, polyquaternium-37, polyquaternium-38, polyquaternium-39, polyquaternium-43, polyquaternium-44, polyquaternium-45, polyquaternium-46, polyquaternium-47, polyquaternium-48, polyquaternium-49, polyquaternium-50, polyquaternium-52, polyquaternium-53, polyquaternium-54, polyquaternium-55, polyquaternium-56, polyquaternium-57, polyquaternium-58, polyquaternium-59, polyquaternium-60, polyquaternium-63, polyquaternium-64, polyquaternium-65, polyquaternium-66, polyquaternium-67, polyquaternium-70, polyquaternium-73, polyquaternium-74, polyquaternium-75, polyquaternium-76, polyquaternium-85, polyquaternium-86, polybeta-alanine, polyepsilonlysine, polylysine, PEG-8/SMDI copolymer, PPG-12/SMDI copolymer, PPG-51/SMDI copolymer, PPG-7/succinic acid copolymer, IPDI/PEG-15 cocamine copolymer, IPDI/PEG-15 cocamine copolymer dimer dilinoleate, IPDI/PEG-15 soyamine copolymer, IPDI/PEG-15 soyamine oxide copolymer, IPDI/PEG-15 soyethonium ethosulfate copolymer, polyquaternium-4/hydroxypropyl starch copolymer, cassia hydroxypropyltrimonium chloride, chitosan hydroxypropyltrimonium chloride, dextran hydroxypropyltrimonium chloride, galactoarabinan hydroxypropyltrimonium chloride, ginseng hydroxypropyltrimonium chloride, guar hydroxypropyltrimonium chloride, hydroxypropyl guar hydroxypropyltrimonium chloride, locust bean hydroxypropyltrimonium chloride, starch hydroxypropyltrimonium chloride, hydroxypropyltrimonium hydrolyzed corn starch, hydroxypropyl oxidized starch PG-trimonium chloride, tamarindus indica hydroxypropyltrimonium chloride, polyacrylamidepropylltrimonium chloride, polymethacrylamidopropylltrimonium chloride, polyacrylamidopropylltrimonium methosulfate, propyltrimoniumchloride methacrylamide/dimethylacrylamide copolymer, acrylamide/ethalkonium chloride acrylate copolymer, acrylamide/ethyltrimonium chloride acrylate/ethalkonium chloride acrylate copolymer, acrylates/carbamate copolymer, adipic acid/methyl DEA crosspolymer, diethylene glycol/DMAP acrylamide/PEG-180/HDI copolymer, dihydroxyethyl tallowamine/IPDI copolymer, dimethylamine/ethylenediamine/epichlorohydrin copolymer, HEMA glucoside/ethylmethacrylate trimonium chloride copolymer, hydrolyzed wheat protein/PEG-20 acetate copolymer, hydrolyzed wheat protein/PVP crosspolymer, ethyltrimonium chloride methacrylate/hydroxyethylacrylamide copolymer and quaternized phosphate esters, i.e. linoleamidopropyl PG-dimonium chloride phosphate; more preferably the at least one component (E) is selected from the group consisting of polyquaternium-10 and guar hydroxypropyltrimonium chloride.

In a preferred embodiment, the at least one component (E) is present in the cleansing composition of the presently claimed invention in an amount in the range of ≥ 0.1 to ≤ 1.0 wt.%, more preferably in the range of ≥ 0.15 to ≤ 0.9 wt.%, even more preferably in the range of ≥ 0.2 to ≤ 0.8, 0.7, 0.6. or 0.5 wt.%, based on the total weight of the cleansing composition.

In a preferred embodiment, the cleansing composition of the presently claimed invention comprises at least one cosmetically acceptable adjuvant (F).

In a preferred embodiment, the cleansing composition of the presently claimed invention comprises at least one component (F) which is selected from the group consisting of preservatives, anti-dandruff agents, electrolytes, pH adjusting agents, perfumes, dyes, sequestering agents, fatty acids, fatty acid esters, dicarboxylic acids, hydrocarbons, vitamin E, vitamin E esters, panthenol, panthenyl ethyl ether, hydrolysed keratins, proteins, peptides, plant extracts, nutrients and anti-hair loss agents.

In a preferred embodiment, preservatives are selected from the group consisting of benzyl alcohol, methyl paraben, propyl paraben, imidazolidinyl urea, sodium benzoate, potassium sorbate, salicylic acid, sodium formate and phenoxyethanol, methylchloroisothiazolinone and methylisothiazolinone.

In a preferred embodiment, anti-dandruff agents that are selected from the group consisting of climbazole, octopirox and zinc pyrithione, salicylic acid, elemental sulfur, selenium dioxide, and the azole antimycotics. In a preferred embodiment, the anti-dandruff agent is present in the cleansing composition of the presently claimed invention in an amount in the range of ≥ 0.1 to ≤ 1.0 wt.%, more preferably in the range of ≥ 0.2 to ≤ 0.9 wt.%, even more preferably in the range of ≥ 0.3 to ≤ 0.8, 0.7, 0.6. or 0.5 wt.%, based on the total weight of the cleansing composition.

In a preferred embodiment, the pH adjusting agents are selected from the group consisting of citric acid and its salts, succinic acid and its salts, phosphoric acid and its salts, sodium hydroxide, and sodium carbonate; more preferably the pH adjusting agent is citric acid and its salts, in particular the pH adjusting agent is citric acid trisodium salt. In a preferred embodiment, the pH adjusting agent is present in an amount in the range of ≥ 0.1 to ≤ 0.5 wt.%, based on the total weight of the cleansing composition.

In a preferred embodiment, the term "electrolyte" refers to an ionic salt which is totally soluble in the cleansing composition of the presently claimed invention at the concentrations used. The electrolyte is preferably selected from the group consisting of alkali salts and ammonium salts, more preferably the electrolyte is an alkali salt such as NaCl or KCI. In a preferred embodiment, the electrolyte is present in an amount in the range of ≥ 0.1 to ≤ 0.3 wt.%, based on the total weight of the cleansing composition.

In a preferred embodiment, the cleansing composition includes a perfume obtained from a natural or a synthetic source. The perfume may be used along with a suitable solvent, diluents or carrier. Perfumes may be added in any conventionally known method, for example, admixing to a composition or blending with other ingredients used to form a composition, in amounts which are found to be useful to increase or impart the desired scent characteristics to cleansing compositions. Perfumes for the present application can be one or more selected from the following non-limiting group of compounds such as essential oils, absolutes, resinoids, resins, concretes, alcohols, aldehydes, ketones, ethers, acids, esters, acetals, ketals, nitriles, including saturated and unsaturated compounds and aliphatic, carbocyclic and heterocyclic compounds.

In a preferred embodiment, the cleansing composition of the presently claimed invention includes dyes which herein include natural dyes and dyes suitable for food, drug and cosmetic applications. These colorants are also known as FD & C, and D&C dyes and lakes and are preferably water-soluble in nature.

In yet another embodiment, the cleansing composition comprises sequestering agents. The term "sequestering agent" as used herein relates to a compound which is capable of bonding or complexing a metal ion between two or more atoms of the compound, thereby neutralizing or controlling harmful effects of such metal ions, wherein holding or bonding of a metal ion is through combination of one or more different types of bonds including coordination and/or ionic bonds. The suitable organic or inorganic sequestering agents are selected from the group consisting of polyols, gluconates, sorbitols, mannitols, carbonates, hydroxamates, catechols, alkanolamines, metal-ion sequestrants, hydroxy-carboxylic acids, aminocarboxylic acids, amino polycarboxylic acids, polyamines, polyphosphates, phosphonic acids, crown ethers, amino acids, polycarboxylic acids, cyclodextrin, phosphonates, polyacrylates or polymeric polycarboxylates and condensed phosphates.

In yet another embodiment, the cleansing composition comprises a fatty acid or esters thereof. Suitable fatty acids include myristic acid, palmitic acid, stearic acid, behenic acid, oleic acid, linoleic acid, and isostearic acid.

In yet another embodiment, the cleansing composition comprises a dicarboxylic acid selected from the group consisting of maleic acid, fumaric acid, itaconic acid, oxalic acid, malonic acid, succinic acid, adipic acid, glutaric acid, malic acid, tartaric acid, galactaric acid, citric acid, oxomalonic acid and ketoglutaric acid.

In yet another embodiment, the cleansing composition comprises hydrocarbons and/or polyterpenes.

In a preferred embodiment, the cleansing composition comprises a protein, more preferably a hydrolyzed cationic or non-cationic protein. Examples of these compounds include hydrolyzed collagens having trimethyl ammonium and trimethyl stearyl ammonium chloride groups, hydrolyzed animal proteins having trimethyl benzyl ammonium groups (benzyltrimonium hydrolyzed animal protein), hydrolyzed proteins having groups of quaternary ammonium on the polypeptide chain. Ceramide type of compounds include a ceramide, a glycoceramide, a pseudoceramide, or a neoceramide.

In a preferred embodiment, the cleansing composition comprises vitamins or their derivatives, such as vitamin B complex, including thiamine, nicotinic acid, biotin, pantothenic acid, choline, riboflavin, vitamin B6, vitamin B12, pyridoxine, inositol, carnitine, vitamins A, C, D, E, K and their derivatives, such as vitamin A palmitate, and pro-vitamins, e.g., panthenol (pro vitamin B5), panthenol triacetate and mixtures thereof.

In a preferred embodiment, suitable antioxidants may be added to facilitate the enhanced shelf-life of the cleansing composition of the presently claimed invention. The antioxidants that can be used include vitamins such as vitamin E, vitamin E acetate, vitamin C, vitamin A, and vitamin D, and derivatives thereof. Additional exemplary antioxidants include but are not limited to propyl, octyl and dodecyl esters of gallic acid, butylated hydroxyanisole (BHA), butylated hydroxytoluene (BHT), and nordihydroguaiaretic acid.

In a preferred embodiment, the cleansing composition comprises a plant extract selected from the group consisting of alnus, arnica, artemisia capillaris, Asia arum root, calendula, chamomile, conidium, comfrey, fennel, hawthorn, houttuynia, hypericum, jujube, kiwi, licorice, magnolia, olive, peppermint, philodendron and salvia.

In a preferred embodiment, the cleansing composition comprises anti-hair loss agents selected from the group consisting of caffeine and sandal pentanol.

In a preferred embodiment, the at least one component (F) is present in an amount in the range of ≥ 0.5 to ≤ 4.0 wt.%, based on the total weight of the cleansing composition.

In a preferred embodiment, the cleansing composition of the presently claimed invention has a pH in the range of ≥ 4.0 to ≤ 6.5; more preferably in the range of ≥ 5.0 to ≤ 6.0.

In a preferred embodiment, the cleansing composition of the presently claimed invention is free of sulfate-containing surfactants, i.e. the composition does not contain (0 %) of any anionic surfactant which is a derivative of a sulfate, such as sodium lauryl sulfate (SLS), sodium laureth sulfate (SLES), ammonium lauryl sulfate (ALS) or ammonium laureth sulfate (ALES), more preferably cleansing composition of the presently claimed invention is free of sodium lauryl ether sulfate and sodium lauryl sulfate.

In a preferred embodiment, the cleansing composition of the presently claimed invention is free of non-ionic surfactants, i.e. the composition does not contain (0 %) non-ionic surfactants. Non-ionic surfactants are selected from the group consisting of fatty alcohol polyoxyalkylene esters, alkyl polyoxyalkylene ethers which are derived from C1-C6-alcohols or from C7-C30-fatty alcohols, alkylaryl alcohol polyoxyethylene ethers, alkoxylated animal and/or plant fats and/or oils, glycerol esters, alkylphenol alkoxylates, fatty amine alkoxylates, fatty acid amide and fatty acid diethanolamide alkoxylates, ethoxylates thereof, sugar surfactants, sorbitol esters, polyoxyethylene sorbitan fatty acid esters, alkyl polyglycosides, N-alkylgluconamides, alkyl methyl sulfoxides and alkyl dimethyl phosphine oxides.

In a preferred embodiment, the cleansing composition of the presently claimed invention is free of non-ionic surfactants selected from the group consisting of alkanolamide surfactants and glycoside surfactants, i.e. the composition does not contain (0 %) non-ionic surfactants selected from the group consisting of alkanolamide surfactants and glycoside surfactants.

Alkanolamide surfactants preferably comprise acetamide MEA, cocamide MEA, cocamide DEA, cocamide methyl MEA, cocamide MIPA, hydroxystearamide MEA, PEG-5 cocamide MEA, lactamide MEA, lauramide MEA and lauramide DEA.

Glycoside surfactants preferably comprise C4-C22 alkylhexosides, such as butylglucoside, nonylglucoside, decylglucoside, dodecylglucoside, hexadecylglucoside, octadecylglucoside, cocoglucoside, laurylglucoside, caproyl ethyl glucoside, caprylyl/capryl glucoside, caprylyl glucoside, C4-C22 alkylpolyhexosides, such as butylpolyglucosides, nonylpolyglucosides, decylpolyglucosides, tetradecylpoly-glucosides, hexadecylpolyglucosides, erucylpolyglucosides, C4-C33 alkylpentosides, such as nonylarbinosides, decylarabinoside, hexadecylarabinoside, octylxyloside, nonylxyloside, decylxyloside, hexadecylxyloside, erucylxyloside and C4-C22 alkylpolypentosides, such as butylpolyarabinosides, nonylpolyarbinosides, decylpolyarabinosides, hexadecylpolyarbinosides, octadecylpolyarabinosides, erucylpolyarabinosides, butylpolyxylosides, nonylpolyxylosides, decylpolyxylosides, octadecylpolyxylosides and erucylpolyxylosides, butylpoly(arabino-co-xylo)sides, nonylpoly(arbino-co-xylo)sides, decylpoly(arbino-co-xylo)sides, hexadecylpoly(arabino-coxylo)sides, octadecylpoly(arabino-co-xylo)sides and erucylpoly(arabino-co-xylo)sides, wherein the term "poly(arabino-co-xylo)side" denotes a copolymeric chain of monomeric residue of arabinose and xylose.

In a preferred embodiment, the total amount of anionic surfactants, i.e., the at least component (A), the at least component (B) and the at least component (C), is greater than the total amount of the amphoteric or zwitterionic surfactant, i.e. the total amount of the at least one component (D); more preferably the ratio of the sum of wt.% of the at least one component (A), the at least one component (B) and the at least one component (C) to the wt.% of the at least one component (D) is in the range of ≥ 1.8:1.0 to ≤ 3.0:1.0.

In a preferred embodiment, the cleansing composition of the presently claimed invention has a viscosity in the range of ≥ 1500 to ≤ 50000 mPa.s, more preferably in the range of ≥ 2500 to ≤ 30000 mPa.s, even more preferably in the range of ≥ 3000 to ≤ 25000 mPa.s, measured after 24-hours in a temperature-controlled room (21 °C) using a Brookfield Viscosimeter Modell DV-II at 10 RPM, with a RV spindle 4 or 5.

In a preferred embodiment, the cleansing composition of the presently claimed invention comprises
(A) ≥ 1.5 to ≤ 5.0 wt.% of at least one taurate surfactant selected from the group consisting of sodium methyl cocoyl taurate and sodium methyl oleoyl taurate;
(B) ≥ 2.0 to ≤ 8.0 wt.% of at least one surfactant selected from the group consisting of sodium cocoyl isethionate and sodium cocoyl methyl isethionate;
(C) ≥ 1.0 to ≤ 4.0 wt.% of at least one N-acylated amino acid surfactant selected from the group consisting of sodium lauroyl sarcosinate;
(D) ≥ 2.0 to ≤ 6.0 wt.% cocoamidopropyl betaine;
(E) ≥ 0.1 to ≤ 1.0 wt.% of at least one conditioning agent selected from the group consisting of polyquaternium-10 and guar hydroxypropyltrimonium chloride;
whereby the wt.% are each based on the total weight of the cleansing composition.

In a preferred embodiment, the cleansing composition of the presently claimed invention is used in a manner known in the art, for example, in the case of a shampoo, by application of the shampoo to the skin and/or hair and optionally rinsing the shampoo off of the skin and/or hair with water. Accordingly, the presently claimed invention is also directed to a method for cleansing hair comprising applying the cleansing composition as described herein to the skin and/or the hair.

In another preferred embodiment, the presently claimed invention is directed to the use of the cleansing composition as described herein for cleansing hair and/or skin.

The presently claimed invention is associated with one or more of the following advantages.
- The cleansing composition of the presently claimed invention exhibits excellent foaming behaviour.
- The cleansing composition of the presently claimed invention shows good conditioning on hair.
- The cleansing composition of the presently claimed invention has a satisfactory viscosity, i.e., a viscosity in the range of ≥ 1500 to ≤ 50000 mPa.s.
- The cleansing composition of the presently claimed invention contains low amounts of surfactants.

### Examples

The presently claimed invention is illustrated in detail by working examples which follow. More particularly, the test methods specified hereinafter are part of the general disclosure of the application .

### Materials

- Sodium methyl oleoyl taurate is commercially available from Innospec Performance Materials (PUREACT MS-CG)
- Sodium methyl cocoyl taurate is commercially available from Innospec Performance Materials (PUREACT MS-WS)
- Sodium lauroyl sarcosinate is commercially available from Croda (Crodasinic LS30 NT MBAC)
- Sodium cocoyl glutamate is commercially available from. BASF (PLANTAPON^{®} ACG 50)
- Sodium cocoyl methyl isethionate is commercially available from Innospec Performance Materials (Iselux^{®} SCMI)
- Cocoamidopropyl betaine is commercially available from Evonik (TEGO^{®} Betain F 50)
- Polyquaternium-10 is commercially available from Dow Chemicals (UCARE^{™} Polymer JR-400)
- Guar hydroxypropyltrimonium chloride is commercially available from Solvay (JAGUAR Excel)
- Citric acid is commercially available from Jungbunzlauer
- Sodium benzoate is commercially available from KALAMA CHEMICALS

### Preparation of shampoos

The exemplified compositions can be prepared by conventional formulation and mixing techniques. All parts, percentages, and ratios herein are by weight unless otherwise specified. Some components may come from suppliers as dilute solutions.

**Table 1**

| | **Composition 1*** | **Composition 2** | **Composition 3** | **Composition 4** | **Composition 5*** | **Composition 6*** |
|---|---|---|---|---|---|---|
| Sodium Methyl Oleoyl Taurate | 2.5 | 2.5 | 2.5 | 2.5 | 2.5 | - |
| Sodium Methyl Cocoyl Taurate | - | - | - | - | - | 2.5 |
| Sodium Lauroyl Sarcosinate | - | 1.5 | 1.5 | 1.5 | - | - |
| Sodium Cocoyl Glutamate | - | - | - | - | 1.5 | 1.5 |
| Sodium Cocoyl Methyl isethionate | 4.8 | 4.8 | 4.8 | - | 4.8 | 4.8 |
| Sodium Cocoyl isethionate | - | - | - | 4.5 | - | - |
| Cocamidopropyl Betaine | 4.5 | 4.5 | 4.5 | 4.5 | 4.5 | 4.5 |
| Polyquaternium-10 | 0.4 | 0.4 | - | 0.4 | 0.4 | 0.4 |
| Guar Hydroxypropyltrimo nium Chloride | - | - | 0.2 | - | - | - |
| Citric Acid** | 0.2 - 0.4 | 0.2 - 0.4 | 0.2 - 0.4 | 0.2 - 0.4 | 0.2 - 0.4 | 0.2 - 0.4 |
| Sodium Benzoate | 0.4 | 0.4 | 0.4 | 0.4 | 0.4 | 0.4 |
| Perfume | 0.5 | 0.5 | 0.5 | 0.5 | 0.5 | 0.5 |
| Water | to 100 | to 100 | to 100 | to 100 | to 100 | to 100 |
| pH | 5-6 | 5-6 | 5-6 | 5-6 | 5-6 | 5-6 |

| | | | | | | |
|---|---|---|---|---|---|---|
| *not within the scope of the presently claimed invention ** Citric Acid level can vary within the given range for pH adjustment. | | | | | | |

### Test methods

### Half head test

Half head tests were carried out by stylists. Half-head comparisons enable the hairstylist to evaluate the effects of hair products in comparison with a defined standard. The half head test involved application of a first sample to the half head of one model, and application of a second sample to the other half head of the same model or comparison to the untreated half head of the same model depending on the styling product and/or styling technique to be evaluated. This test is termed a half-head comparison because the test samples were applied to only one half of the head which enabled a direct comparison under absolutely identical test conditions (identical hair structure, degree of damage, hair colour etc.). The performance of the sulfate free shampoo according to the presently claimed invention (compositions 2 to 4) was compared to the performance of the composition 1 that did not contain any acid-based surfactants.

To demonstrate the benefits of Sodium Lauroyl Sarcosinate, samples of sulfate-free shampoo with different concentrations of Sodium Lauroyl Sarcosinate and without this anionic surfactant were formulated and evaluated with regards to product performance and stability.

Composition 2-2 equals basically composition 2, composition 1-2 and 2-3 are the examples with no/0% and 10% Sodium Lauroyl Sarcosinate respectively:

**Table 2: compositions**

| **Raw Material** | **Composition 2-2** | **Composition 1-2** | **Composition 2-3** |
|---|---|---|---|
| | **wt%** | **wt%** | **wt%** |
| Sodium Methyl Oleoyl Taurate | 2.5 | 2.5 | 2.5 |
| Sodium Lauroyl Sarcosinate | 1.5 | 0 | 10 |
| Sodium Cocoyl Methyl Isethionate | 4.8 | 4.8 | 4.8 |
| Cocamidopropyl Betaine | 4.5 | 4.5 | 4.5 |
| Disodium EDTA | 0.16 | 0.16 | 0.16 |
| Sodium Benzoate | 0.5 | 0.5 | 0.5 |
| Citric Acid Anhydrous | 0.2 | 0.2 | 0.2 |
| Phenoxyethanol and Ethylhexylglycerin | 0.7 | 0.7 | 0.7 |
| Disodium EDTA | 0.16 | 0.16 | 0.16 |
| Sodium Xylenesulfonate | 0.00001 | 0.00001 | 0.00001 |
| Water | to 100 | to 100 | to 100 |

The impact of Sodium Lauroyl Sarcosinate on the product performance and stability of the invented cleansing composition is examined by half head comparisons and viscosity/rheology measurements. The half-head test was carried out as described above.

### Evaluation Criteria for Half Head Examination

### Foaming Pre-Wash

Half of each of the shampoo amounts is applied in dots on the two sides. and then distribute with even massaging movements. Lather ability is judged upon the first 30 seconds of lathering and evaluated as easier/more difficult.

### Foaming Main-Wash

Repeat the shampoo procedure once more. as described above. Squeeze foam from the middle to the hair tips of each section. Transfer foam from each hands on the top (crown) area. Observe which side has more /less amount of foam.

### Feel of Foam

During main wash step, gently press foam from each section with palms and rub foam with fingertips to assess the thickness which foam is more creamy or more watery.

### Lather Quality

Squeeze foam from the top area to the hair tips of each section again. Obverse foam bubbles on the back of hands, the size of the lather bubbles is assessed visually. Small bubbles = small pores, large bubbles = large pores

### Wet and Dry Combability

Combability of the hair is assessed by placing an aluminum comb parallel to the middle parting and running it through the hair to the shoulder. The comb must remain at a 90° angle throughout and remain in contact with the scalp throughout combing to avoid varying comb angles. The amount of resistance/effort needed during combing is the basis for evaluating the product as easier to comb/more difficult to comb. This test can be carried out on wet hair and on dry hair as well. The evaluation is conducted manually and is compared to the combability of the hair with reference conditioner.

### Anti-Frizz

Hair Frizz is evaluated optically - on straight hair styles via assessing if short strands sticking up at the part and throughout the hair length that "project away from the main body of hair", and on curly hair via assessing how much strands of wavy or curly hair do not align with others to form a defined wave or curl. The less hair sticking up on straight hair and the more defined/less unruly curls or waves are observed, the less frizzy the hair is.

### Volume/Body

The hair is combed in the direction of the style, using a wide-toothed comb, and the model shakes her head twice. Standing about 1m behind the model, the 'volume' is then evaluated visually as more/less volume, i.e. more or less distance away from head.

Half head examination was carried out with 5 test persons, the numbers indicates how many test persons were judged for each criterion.

**Table 3: half head test results**

| Criterion | Composition 2-2 (with 1.5% Sodium Lauroyl Sarcosinate) better than composition 1-2 (without Sodium Lauroyl Sarcosinate) | Composition 2-2 (with 1.5% Sodium Lauroyl Sarcosinate) equal to composition 1-2 (without Sodium Lauroyl Sarcosinate) | Composition 2-2 (with 1.5% Sodium Lauroyl Sarcosinate) worse than composition 1-2 (without Sodium Lauroyl Sarcosinate) |
|---|---|---|---|
| Foaming Pre-Wash | 2 | 3 | 0 |
| Foaming Main-Wash | 3 | 2 | 0 |
| Feel of Foam | 4 | 1 | 0 |
| Lather Quality | 3 | 2 | 0 |
| Wet Combability | 2 | 2 | 1 |
| Dry Combability* | 1 | 3 | 0 |
| Volume/Body | 2 | 1 | 2 |
| Anti-Frizz | 1 | 1 | 3 |

| | | | |
|---|---|---|---|
| * Dry combability half head tests were carried out among four test persons/panelists | | | |

The results of the half head examination (table 3) indicate clearly that the Sodium Lauroyl Sarcosinate enhances the foaming performance (on 60% of the panelists a foaming improvement was found: Foaming Pre-Wash, Foaming Main-Wash, Feel of Foam, Lather Quality) whereas the hair conditioning performance (Wet Combability, Dry Combability, Volume/Body, Anti-Frizz) was not impacted (no difference between the five half head tests on average).

Therefore, Sodium Lauroyl Sarcosinate surprisingly enhances the foaming performance within the invented composition, even at a relatively low concentration (1.5%).

### Viscosity

Viscosity of compositions 1-2, 2-2 and 2-3 were measured with a Brookfield R/S PLUS Rheometer, at 26.7 °C, shear rate 2 s⁻¹, cone C75-1, sample volume 2.5 ml. Results:

**Table 4: viscosity results**

| | Composition 2-2 (with 1.5% Sodium Lauroyl Sarcosinate) | Composition 1-2 (with no/0% Sodium Lauroyl Sarcosinate) | Composition 2-3 (with 10% Sodium Lauroyl Sarcosinate) |
|---|---|---|---|
| Viscosity / mPas: | 3090 (ideal shampoo viscosity) | 21735 (far too viscous/thick) | 685 (too runny viscosity) |

The viscosity data indicated a surprisingly strong impact of the Sodium Lauroyl Sarcosinate concentration on the viscosity of the invented composition - the viscosity of the invented shampoos is far too high/too thick with 10% Sodium Lauroyl Sarcosinate (21735 mPas) and too low/too thin with no/0% Sodium Lauroyl Sarcosinate.

A relatively low concentration (1.5%) of Sodium Lauroyl Sarcosinate delivers the optimal shampoo viscosity.

### Conclusions

A low Sodium Lauroyl Sarcosinate concentration in the invented shampoo composition does surprisingly result in an optimal shampoo viscosity with no need to add additional thickeners respectively viscosity adjusters and provides a certain shampoo foaming boost as well.

The Sarcosinate seems to co-aggregate with the other surfactants of the invented composition which is beneficial to form micelle aggregates of a size required to achieve the typical shampoo viscosity. The surfactant colloids of the invented shampoo without Sarcosinate are too small, the viscosity remains too low/runny. On the other hand, an higher Sarcosinate concentration of 10% results in too high viscosity/ a too viscous shampoos which cannot be used as a shampoo either.

## Claims

1. A cleansing composition comprising
(A) ≥ 1.0 to ≤ 8.0 wt.% of at least one taurate surfactant;
(B) ≥ 1.0 to ≤ 10.0 wt.% of at least one surfactant selected from the group consisting of isethionate surfactants and methyl isethionate surfactants;
(C) ≥ 0.5 to ≤ 5.0 wt.% of at least one N-acylated amino acid surfactant; and
(D) ≥ 1.0 to ≤ 8.0 wt.% of at least one betaine;
whereby the wt.% are each based on the total weight of the cleansing composition, and wherein the at least one N-acylated amino acid surfactant is selected from the group consisting of sodium lauroyl sarcosinate, sodium cocoyl sarcosinate, sodium myristoyl sarcosinate, sodium oleoyl sarcosinate, and ammonium lauroyl sarcosinate.

2. The cleansing composition according to claim 1, wherein the at least one taurate surfactant (A) is selected from the group consisting of sodium methyl cocoyl taurate and sodium methyl oleoyl taurate.

3. The cleansing composition according to claim 1 or 2, wherein the at least one component (A) is present in an amount in the range of ≥ 1.5 to ≤ 5.0 wt.%, based on the total weight of the cleansing composition.

4. The cleansing composition according to any one of claims 1 to 3, wherein the at least one surfactant (B) is selected from the group consisting of sodium cocoyl isethionate and sodium cocoyl methyl isethionate.

5. The cleansing composition according to any one of claims 1 to 4, wherein the at least one component (B) is present in an amount in the range of ≥ 2.0 to ≤ 8.0 wt.%, based on the total weight of the cleansing composition.

6. The cleansing composition according to any one of claims 1 to 5, wherein the at least one N-acylated amino acid surfactant (C) is selected from the group consisting of sodium lauroyl sarcosinate.

7. The cleansing composition according to any one of claims 1 to 6, wherein the at least one component (C) is present in an amount in the range of ≥ 1.0 to ≤ 4.0 wt.%, based on the total weight of the cleansing composition.

8. The cleansing composition according to any one of claims 1 to 7, wherein the at least one betaine (D) is cocoamidopropyl betaine.

9. The cleansing composition according to any one of claims 1 to 8, wherein the at least one component (D) is present in an amount in the range of ≥ 2.0 to ≤ 6.0 wt.%, based on the total weight of the cleansing composition.

10. The cleansing composition according to any one of claims 1 to 9, wherein the cleansing composition has a pH in the range of ≥ 5.0 to ≤ 6.0.

11. The cleansing composition according to any one of claims 1 to 10, wherein the cleansing composition is free of sodium lauryl ether sulfate and sodium lauryl sulfate.

12. The cleansing composition according to any one of claims 1 to 11, wherein the cleansing composition is free of non-ionic surfactants.

13. The cleansing composition according to any one of claims 1 to 12 wherein the ratio of the sum of wt.% of the at least one component (A), the at least one component (B) and the at least one component (C) to the wt.% of the at least one component (D) is in the range of ≥ 1.8:1.0 to ≤ 3.0:1.0.

14. A method for cleansing hair comprising applying the cleansing composition of any one of claims 1 to 13 to the hair.

15. The use of a cleansing composition according to any one of claims 1 to 13 for cleansing hair.

## Patentansprüche

1. Eine Reinigungszusammensetzung, umfassend
(A) ≥ 1,0 bis ≤ 8,0 Gew.-% mindestens eines Taurattensids;
(B) ≥ 1,0 bis ≤ 10,0 Gew.-% mindestens eines Tensids, ausgewählt aus der Gruppe bestehend aus Isethionattensiden und Methylisethionattensiden;
(C) ≥ 0,5 bis ≤ 5,0 Gew.-% mindestens eines N-acylierten Aminosäuretensids; und
(D) ≥ 1,0 bis ≤ 8,0 Gew.-% mindestens eines Betains;
wobei sich die Gewichtsprozente jeweils auf das Gesamtgewicht der Reinigungszusammensetzung beziehen und wobei das mindestens eine N-acylierte Aminosäuretensid aus der Gruppe ausgewählt ist, die aus Natriumlauroylsarcosinat, Natriumcocoylsarcosinat, Natriummyristoylsarcosinat, Natriumoleoylsarcosinat und Ammoniumlauroylsarcosinat besteht.

2. Reinigungszusammensetzung nach Anspruch 1, wobei das mindestens eine Taurattensid (A) aus der Gruppe ausgewählt ist, die aus Natriummethylcocoyltaurat und Natriummethyloleoyltaurat besteht.

3. Reinigungszusammensetzung nach Anspruch 1 oder 2, wobei die mindestens eine Komponente (A) in einer Menge im Bereich von ≥ 1,5 bis ≤ 5,0 Gew.-%, bezogen auf das Gesamtgewicht der Reinigungszusammensetzung, vorhanden ist.

4. Reinigungszusammensetzung nach einem der Ansprüche 1 bis 3, wobei das mindestens eine Tensid (B) aus der Gruppe ausgewählt ist, die aus Natriumcocoylisethionat und Natriumcocoylmethylisethionat besteht.

5. Reinigungszusammensetzung nach einem der Ansprüche 1 bis 4, wobei die mindestens eine Komponente (B) in einer Menge im Bereich von ≥ 2,0 bis ≤ 8,0 Gew.-%, bezogen auf das Gesamtgewicht der Reinigungszusammensetzung, vorhanden ist.

6. Reinigungszusammensetzung nach einem der Ansprüche 1 bis 5, wobei das mindestens eine N-acylierte Aminosäuretensid (C) aus der Gruppe ausgewählt ist, die aus Natriumlauroylsarcosinat besteht.

7. Reinigungszusammensetzung nach einem der Ansprüche 1 bis 6, wobei die mindestens eine Komponente (C) in einer Menge im Bereich von ≥ 1,0 bis ≤ 4,0 Gew.-%, bezogen auf das Gesamtgewicht der Reinigungszusammensetzung, vorhanden ist.

8. Reinigungszusammensetzung nach einem der Ansprüche 1 bis 7, wobei das mindestens eine Betain (D) Cocoamidopropylbetain ist.

9. Reinigungszusammensetzung nach einem der Ansprüche 1 bis 8, wobei die mindestens eine Komponente (D) in einer Menge im Bereich von ≥ 2,0 bis ≤ 6,0 Gew.-%, bezogen auf das Gesamtgewicht der Reinigungszusammensetzung, vorhanden ist.

10. Die Reinigungszusammensetzung gemäß einem der Ansprüche 1 bis 9, wobei die Reinigungszusammensetzung einen pH-Wert im Bereich von ≥ 5,0 bis ≤ 6,0 aufweist.

11. Reinigungszusammensetzung nach einem der Ansprüche 1 bis 10, wobei die Reinigungszusammensetzung frei von Natriumlaurylethersulfat und Natriumlaurylsulfat ist.

12. Die Reinigungszusammensetzung gemäß einem der Ansprüche 1 bis 11, wobei die Reinigungszusammensetzung frei von nichtionischen Tensiden ist.

13. Reinigungszusammensetzung nach einem der Ansprüche 1 bis 12, wobei das Verhältnis der Summe der Gewichtsprozente der mindestens einen Komponente (A), der mindestens einen Komponente (B) und der mindestens einen Komponente (C) zu den Gewichtsprozenten der mindestens einen Komponente (D) im Bereich von ≥ 1,8 : 1,0 bis ≤ 3,0 : 1,0 liegt.

14. Verfahren zum Reinigen von Haaren, bei dem man die Reinigungszusammensetzung nach einem der Ansprüche 1 bis 13 auf das Haar aufträgt.

15. Verwendung einer Reinigungszusammensetzung gemäß einem der Ansprüche 1 bis 13 zur Reinigung von Haaren.

## Revendications

1. Une composition nettoyante comprenant
(A) ≥ 1,0 à ≤ 8,0 % en poids d'au moins un tensioactif taurate;
(B) ≥ 1,0 à ≤ 10,0 % en poids d'au moins un tensioactif choisi dans le groupe constitué par les tensioactifs iséthionate et les tensioactifs méthyliséthionate;
(C) ≥ 0,5 à ≤ 5,0 % en poids d'au moins un tensioactif d'acide aminé N-acylé; et
(D) ≥ 1,0 à ≤ 8,0 % en poids d'au moins une bétaïne;
dans lequel les % en poids sont chacun basés sur le poids total de la composition nettoyante, et dans lequel le ou les tensioactifs d'acide aminé N-acylé sont choisis dans le groupe constitué par le lauroyl sarcosinate de sodium, le cocoyl sarcosinate de sodium, le myristoyl sarcosinate de sodium, l'oléoyl sarcosinate de sodium et le lauroyl sarcosinate d'ammonium.

2. Composition nettoyante selon la revendication 1, dans laquelle le ou les tensioactifs taurate (A) sont choisis dans le groupe constitué par le méthyl cocoyl taurate de sodium et le méthyl oléoyl taurate de sodium.

3. Composition nettoyante selon la revendication 1 ou 2, dans laquelle au moins un composant (A) est présent en une quantité comprise dans la plage de ≥ 1,5 à ≤ 5,0 % en poids, par rapport au poids total de la composition nettoyante.

4. Composition nettoyante selon l'une quelconque des revendications 1 à 3, dans laquelle le ou les tensioactifs (B) sont choisis dans le groupe constitué par le cocoyl iséthionate de sodium et le cocoyl méthyl iséthionate de sodium.

5. Composition nettoyante selon l'une quelconque des revendications 1 à 4, dans laquelle le ou les composants (B) sont présents en une quantité comprise dans la plage de ≥ 2,0 à ≤ 8,0 % en poids, par rapport au poids total de la composition nettoyante.

6. Composition nettoyante selon l'une quelconque des revendications 1 à 5, dans laquelle le ou les tensioactifs d'acides aminés N-acylés (C) sont choisis dans le groupe constitué par le lauroyl sarcosinate de sodium.

7. Composition nettoyante selon l'une quelconque des revendications 1 à 6, dans laquelle le ou les composants (C) sont présents en une quantité comprise dans la plage de ≥ 1,0 à ≤ 4,0 % en poids, par rapport au poids total de la composition nettoyante.

8. Composition nettoyante selon l'une quelconque des revendications 1 à 7, dans laquelle la ou les bétaïnes (D) sont la bétaïne de cocamidopropyle.

9. Composition nettoyante selon l'une quelconque des revendications 1 à 8, dans laquelle le ou les composants (D) sont présents en une quantité comprise entre ≥ 2,0 et ≤ 6,0 % en poids, par rapport au poids total de la composition nettoyante.

10. Composition nettoyante selon l'une quelconque des revendications 1 à 9, dans laquelle la composition nettoyante a un pH dans la plage de ≥ 5,0 à ≤ 6,0.

11. Composition nettoyante selon l'une quelconque des revendications 1 à 10, dans laquelle la composition nettoyante est exempte de lauryl éther sulfate de sodium et de lauryl sulfate de sodium.

12. Composition nettoyante selon l'une quelconque des revendications 1 à 11, dans laquelle la composition nettoyante est exempte de tensioactifs non ioniques.

13. Composition nettoyante selon l'une quelconque des revendications 1 à 12, dans laquelle le rapport de la somme du % en poids du ou des composants (A), du ou des composants (B) et du ou des composants (C) au % en poids du ou des composants (D) est compris dans la plage de ≥ 1,8 : 1,0 à ≤ 3,0 : 1,0.

14. Procédé de nettoyage des cheveux comprenant l'application de la composition nettoyante selon l'une quelconque des revendications 1 à 13 sur les cheveux.

15. Utilisation d'une composition nettoyante selon l'une quelconque des revendications 1 à 13 pour nettoyer les cheveux.
